# EUROPEAN PATENT SPECIFICATION

(11) **EP 1 738 171 B1**
(45) Date of publication and mention of the grant of the patent: **08.04.2009**
(21) Application number: 05736155.2
(22) Date of filing: 18.04.2005
(51) Int. Cl.: G01N 33/542, C12Q 1/34

(54) **FLUORESCENCE RESONANCE ENERGY TRANSFER ENZYME SUBSTRATES**
ENZYMSUBSTRATE FÜR FLUORESZENZRESONANZ-ENERGIETRANSFER
SUBSTRATS DE TRANSFERT D'ENERGIE PAR RESONANCE A FLUORESCENCE

(30) Priority: 23.04.2004 US 564924 P
(43) Date of publication of application: 03.01.2007
(73) Proprietor: GE HEALTHCARE AS, Nydalen 0401 Oslo (NO)
(72) Inventor: KUMAR, Shiv, Belle Mead, New Jersey 08502 (US); SOOD, Anup, Clifton Park, New York 12065 (US)
(74) Representative: Canning, Lewis R.
(86) International application number: PCT/US2005/013141
(87) International publication number: WO 2005/108994

(56) References cited:
- US-A- 5 795 729
- US-A1- 2003 186 348
- WAHLER D ET AL: "High-throughput screening for biocatalysts." CURRENT OPINION IN BIOTECHNOLOGY. DEC 2001, vol. 12, no. 6, December 2001 (2001-12), pages 535-544, XP002345702 ISSN: 0958-1669
- GERSHKOVICH ALEXANDER A ET AL: "Fluorogenic substrates for proteases based on intramolecular fluorescence energy transfer (IFETS)" JOURNAL OF BIOCHEMICAL AND BIOPHYSICAL METHODS, vol. 33, no. 3, 1996, pages 135-162, XP002345703 ISSN: 0165-022X

## Description

### Field of Invention

The present invention relates to fluorescence-based assays; and to reagents and methods for measuring enzyme activity, particularly enzyme cleavage assays.

### Background of Invention

Assays for measuring enzyme activity, particularly enzyme cleaving activity such as hydrolysis, are widely employed in the biological and pharmaceutical sciences. With the advent of combinatorial chemistry and high throughput screening, there is a growing need for simple, sensitive and cost-effective assays to screen for potential modulators of enzyme activity. Of particular interest to the pharmaceutical industries are methods for detecting proteolytic enzyme cleavage and phosphate cleavage.

Fluorescence-based assays offer significant advantages over radiochemical, ELISA, antibody and more traditional techniques for measuring enzyme cleaving activity in terms of simplicity of handling, sensitivity, cost and ease of automation. Fluorogenic substrates are routinely used for homogeneous enzyme assays to determine enzyme activity, or the effect of potential drugs on enzyme activity in drug discovery. A number of these enzyme substrates are commercially available, or have been reported in the literature. For example, US 4812409 (Babb, B., et al) discloses hydrolysable fluorescent substrates comprising blocked dye moieties derived from phenalenone or benzphenalenone, which when cleaved from the substrate during enzymatic hydrolysis, form fluorescent dyes having a fluorescence emission above about 530nm. Substrates responsive to hydrolytic enzymes are also available based on derivatives of fluorescein and rhodamine. For example, fluorescein diphosphate is a widely used, non-fluorescent and colourless substrate for alkaline phosphatase (PP2A), which when hydrolysed by this enzyme forms fluorescein (Vieytes M., et al, Anal. Biochem., (1997), 248, 258-64). However, substrates based on fluorescein usually incorporate two enzyme cleavable sites, and bi-phasic enzyme kinetics takes place through cleavage, firstly to a mono-substituted fluorescent analogue, then to the free fluorophore. Because the mono-substituted fluorescent substrate absorbs and emits at the same wavelength as the free fluorophore, interpretation of the enzyme kinetics is more difficult. Fluorogenic substrates based on fluorinated derivatives of 4-methylumbelliferone have been described for the assay of β-galactosidase activity and acid phosphatase activity (Gee, K.R., et al, (1999), 273, 41-8).

US 5795729 and US 2003/0186348 disclose fluorescent dual enzyme substrates of formula M-D₁-L-D₂, wherein D₁ represents the acceptor in a fluorescence resonance energy transfer arrangement, D₂ represents the corresponding donor, M represents a substrate for a first enzyme and is able to modulate the fluorescence properties of D₁, and L comprises an enzyme cleavable group which is a substrate for a second, different enzyme.

Many of the dyes used in fluorescence based enzyme assays, emit in the 350-550 nm range. Some of the dyes that do emit at higher wavelength, e.g. resorufin and DDAO are reactive towards thiols (including peptides/proteins with thiol functionalities) and lose their fluorescence properties. They are therefore unlikely to be useful in *in vivo* applications. Fluorescence-based enzyme substrates that have dyes with useful fluorescence properties that emit in the red or infrared region of the spectrum are nitro-substituted cyanine dye derivatives (EP 1086179 B1: Hamilton, A. et al). The fluorescence of these dyes is quenched by the presence in the molecule of a substituted nitro group. Upon reduction of the nitro group by a nitroreductase, the dye becomes fluorescent. These fluorogenic substrates have proved to be useful and are currently used in live cell assays; however, they have only limited applications. Hence, there is a clear need for new fluorogenic enzyme substrates with emission at higher wavelength (red or infra-red) that are non-reactive except towards the enzyme of interest and provide a high signal to background ratio.

The present invention describes new fluorogenic enzyme substrates that are substrates for a range of different enzymes and emit at different wavelengths including red and near infra-red. These substrates are dual enzyme substrates for determining the ratios of two enzyme activities. This is more relevant for *in vivo* systems, where two separate enzyme substrates cannot be relied upon to measure relative enzyme activities, due to differences in absorption, distribution and excretion properties of individual single enzyme substrates. Finally, also provided are methods for determining enzyme activity, as well as methods that are useful for determining a metabolic state either *in vitro* or *in vivo.*

It is thus an object of the invention to provide a fluorescence-labelled dual enzyme substrate, particularly a fluorescence resonance energy transfer (FRET) labelled substrate and a method of measuring the activity of two enzymes in cleaving a substrate, the substrate comprising a FRET label, an enzyme cleavable group and an enzyme cleavable linker.

It is also an object of the invention to provide a method of screening for a test agent which may affect enzyme cleaving activity.

### Summary of Invention

Thus, in a first aspect of the invention, there is provided a compound of formula (I):

M-D₁-L-D₂ (I)

wherein:
D₁ is a first dye moiety whose fluorescence properties may be modulated so as to be suitable as a donor in an energy transfer arrangement, wherein D₁ is capable of transferring energy to D₂ and is selected from: coumarin dyes, benzocoumarin dyes, acridone dyes, xanthine dyes, phenoxazine dyes, rhodamine dyes, merocyanine dyes and cyanine dyes, preferably xanthine dyes and cyanine dyes;
D₂ is a second dye moiety suitable as an acceptor in an energy transfer arrangement with said first dye and is selected from: coumarin dyes, benzocoumarin dyes, acridone dyes, xanthine dyes, phenoxazine dyes, rhodamine dyes, merocyanine dyes and cyanine dyes;
M is an enzyme cleavable group chosen to modulate the fluorescence properties of D₁, wherein M comprises a substrate for a first cleavage enzyme;
L is a linking group comprising 2-200 linked atoms, wherein said linking group is a cleavable linker and includes an enzyme cleavage group P which is a substrate for a second cleavage enzyme different from said first cleavage enzyme.

D₁ and D₂ are linked by linking group L such that, under suitable conditions, fluorescence resonance energy transfer (FRET) may take place one with the other. FRET is a distance-related process in which the electronic excited states of two dye molecules interact without emission of a photon. See, Forster, T., "Intermolecular Energy Transfer and Fluorescence", Ann. Physik., Vol.2, p.55, (1948). One result of this interaction is that excitation of a donor molecule enhances the fluorescence emission of an acceptor molecule. The fluorescence quantum yield of the donor is correspondingly diminished. For FRET to occur, suitably, the donor and acceptor dye molecules must be in close proximity (typically between 10-100 Å), since energy transfer efficiency decreases inversely as the 6^{th} power of the distance (r) between the donor and acceptor molecules. In the present invention, D1 is the donor molecule and D₂ is the acceptor in the FRET relationship. By donor, it is meant that the dye moiety is capable of absorbing energy from light and emits light at wavelength frequencies which are at least partly within the absorption spectrum of the acceptor. By acceptor, it is meant that the dye moiety is capable of absorbing energy at a wavelength emitted by a donor dye moiety. There is overlap between at least a portion of the emission spectrum of the donor dye molecule with the absorption spectrum of the acceptor dye molecule. The wavelength of the emission maximum of the acceptor dye is longer than the wavelength of the emission maximum of the donor dye. In one format, one of the donor or acceptor molecules can be a non-fluorescent (or quenching) fluorophor which is in close proximity to a second fluorescent acceptor or donor fluorophor. Upon excitation of the non-fluorescent dye, energy is dissipated as heat rather than fluorescence energy and resonance energy transfer or fluorescence emission cannot take place.

Group M is chosen from enzyme cleavable groups that are capable of modulating the fluorescence properties of D₁, such that when M is covalently attached to D₁, D₁ is in a first fluorescence state, either having no, or substantially no fluorescence emission, or alternatively having a fluorescence emission with an emission wavelength maximum, Eₘₐₓ = λ1'.

M comprises a substrate for an enzyme, such that upon enzymatic cleavage of M from D₁ to form the compound of formula (A'), (see Figure 1), the fluorescence properties of D₁ are caused to be changed to a second fluorescence state such that D₁ emits fluorescence at an emission wavelength maximum, Eₘₐₓ = λ1. Suitably, D₂ is a fluorescent dye having a fluorescence emission maximum, Eₘₐₓ = λ2. Suitably, in the compound of formula (I), λ1' ≠ λ1 ≠ λ2.

Suitably, linking group L comprises a group containing from 2-200 linked atoms selected from carbon atoms which include one or more groups selected from -C(O)-, -NR'-, -O-, -S-, -CH=CH-, -CO-NH-, phenylenyl and the group: 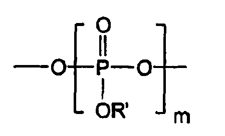 where R' is selected from hydrogen and C₁ - C₄ alkyl and m is an integer from 1 to 3.

In one preferred embodiment, linking group L contains from 2-50 linked atoms, having the structure:

-{(CHR')ₚ-Q-(CHR')ᵣ}ₛ-

where Q is selected from: -C(O)-, -NR'-, -O-, -S-, -CH=CH-, -CO-NH- and phenylenyl groups; R' is hydrogen or C₁ - C₄ alkyl, each p is independently 0 - 10, each r is independently 0 - 10, and s is 1, 2, or 3.

Preferably Q is selected from: -C(O)-, -CHR'-, -O- and -CO-NH-, where R', p, r and s are hereinbefore defined.

In another embodiment, linker group L may also comprise a peptide or an oligonucleotide fragment. When the linker is a peptide or contains a peptide fragment, it may contain from 2-20 natural or unnatural amino acids or a combination thereof. More preferably, L contains 2-12 amino acids and most preferably, 2-8 amino acids. When the linker group L is an oligonucleotide or contains an oligonucleotide fragment, it may contain from 2-20 nucleoside bases or modified bases. Preferably the number of bases will be in the range from 4-15; more preferably in the range 4-10.

The linker may include part of the constituents extending from the fluorochrome. In other words, the linker is attached to the dye chromophore but is not a part of it. In the compounds of the present invention, none of the linkers includes a network of double bonds that permit conjugation of the donor and acceptor. For optimal energy transfer to occur, the transition moments of the donor and acceptor fluorochromes are orientated relative to each other in a non-perpendicular direction, e.g. positioned generally parallel or in tandem relative to each other. With a relatively short linker and optimal orientation, there may be efficient resonance energy transfer even when the spectral overlap becomes small.

### Brief Description of the Figures

Figure 1A is a schematic diagram illustrating a preferred embodiment of the invention. Figure 1B illustrates a reference example.
Figure 2 illustrates the synthetic reaction scheme for the preparation of Compounds (V) and (VI).
Figure 3 shows the UV spectrum of Compound (II) before and after treatment with alkaline phosphatase.
Figure 4 shows the UV spectrum of Compound (III) before and after treatment with alkaline phosphatase.
Figure 5 shows the UV spectrum of Compound (IV) before and after treatment with alkaline phosphatase.
Figure 6 shows the emission spectrum of Compound (II) before and after treatment with Alkaline Phosphatase by excitation at 455 nm and 475 nm.
Figure 7 shows the emission spectrum of Compound (III) before and after treatment with Alkaline Phosphatase by excitation at 455 nm and 475 nm.
Figure 8 shows the emission spectrum of Compound (IV) before and after treatment with Alkaline Phosphatase by excitation at 455 nm and 475 nm.

### Detailed Description of the Preferred Embodiments

According to the first aspect of the invention, D₁ is the donor dye and D₂ is the acceptor dye. In this embodiment (shown in Figure 1A), the emission wavelength maximum (A1) of D₁ is less that the emission wavelength maximum (λ2) of D₂ and there is overlap between at least a portion of the emission spectrum of D₁ with the absorption spectrum of D₂. When M is covalently attached to D1, D₁ has either no, or substantially no fluorescence emission, or alternatively fluoresces having a fluorescence emission wavelength maximum λ1', where λ1' is less than λ1. In this state, there is no, or substantially no, overlap between the emission spectrum of D₁ and the absorption spectrum of D₂, such that there is no energy transfer between D₁ and D₂. Upon cleavage of M from D₁, the fluorescence properties of D₁ are caused to be changed to a second fluorescence state such that D₁ emits fluorescence at the emission wavelength maximum λ1. As a result, overlap between the emission spectrum of the D₁ and the absorption spectrum of D₂ is restored and energy transfer between D₁ and D₂ can take place. Thus, upon excitation of the donor dye D₁ at an appropriate excitation wavelength, an increase in fluorescence emission from D₂ may be detected and/or quantitated.

In a reference example D₂ is the donor dye and D₁ is the acceptor dye. In this example (shown in Figure 1B), the emission wavelength maximum (λ2) of D₂ is less that the emission wavelength maximum (λ1) of D₁ and there is overlap between at least a portion of the emission spectrum of the D₂ with the absorption spectrum of D₁. When M is covalently attached to D₁, D₁ has either no, or substantially no fluorescence emission, or alternatively fluoresces having a fluorescence emission wavelength maximum λ1', where λ1' is greater than λ1. In this state, there is no, or substantially no, overlap between the emission spectrum of D₂ and the absorption spectrum of D₁, such that there is no energy transfer between D₂ and D₁. Upon cleavage of M from D₁, the fluorescence properties of D₁ are caused to be changed to a second fluorescence state such that D₁ emits fluorescence at an emission wavelength maximum λ1. As a result, overlap between the emission spectrum of the D₂ and the absorption spectrum of D₁ is restored and energy transfer can take place. Upon excitation of the donor dye D₂ at an appropriate excitation wavelength, an increase in fluorescence emission from D₁ may be detected and/or quantitated.

In the reference example in which D₂ is the donor dye and D₁ is the acceptor, energy transfer may alternatively take place between the donor dye and two fluorescent states of the acceptor dye. In this arrangement, the acceptor dye in the first fluorescent state will emit at one wavelength λ1' and upon cleavage of M will generate a second fluorescence state that emits at a second wavelength λ1.

In a further embodiment, the first and second fluorescence states of D₁ may differ, not by their respective emission wavelength maxima λ1 and λ1', but by the intensity of fluorescence emitted by the two states.

Compounds of formula (I) may be used as reporter molecules for detecting biochemical cleavage events in assays that employ fluorescence resonance energy transfer. Group M comprises a substrate for a cleavage enzyme, preferably selected from the group consisting of a peptidase, a protease, a phosphatase, a dealkylase and a glycosidase. Treatment of the substrate with an enzyme under conditions suitable to cause cleavage of M from D₁ modulates the fluorescence properties of D₁ thereby switching D₁ from the first fluorescent state to the second fluorescent state as described hereinbefore.

In one preferred embodiment, M comprises a phosphate ester linkage having one or more phosphate groups covalently bonded to D₁ and having the structure: 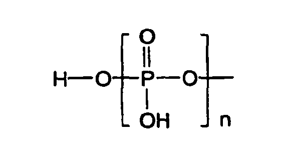 wherein n is an integer from 1 to 4. In this embodiment, the substrate is capable of being cleaved by a phosphatase such as bacterial alkaline phosphatase, or acid phosphatase, to yield an alcohol derivative of dye D₁, and inorganic phosphate. As a consequence, D₁ is caused to be switched from a first fluorescent state to a second fluorescent state, thereby enabling transfer of excitation energy between donor and acceptor moieties. Upon excitation of the donor dye at its excitation wavelength, there is an increase in fluorescence intensity at the emission wavelength of the acceptor dye. The phosphate ester may be a pre-synthesised substrate or may be generated *in situ* by chemical hydrolysis or by an enzyme catalysed nucleoside monophosphate or nucleoside polyphosphate transfer from a terminal-phosphate labelled nucleoside polyphosphate having the structure: 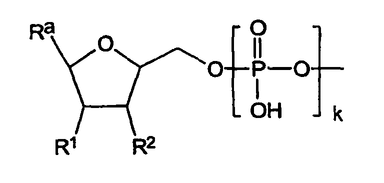 wherein R¹ and R² are independently selected from H and OH; Rₐ is a nucleoside base selected from adenine, guanine cytosine, thymine, uracil, hypoxanthine and xanthine; and k is an integer from 1 to 6.

In another preferred embodiment, M comprises at least one peptide linkage (-CO-NH-) covalently bonded to D₁. In this embodiment, M typically has the structure: 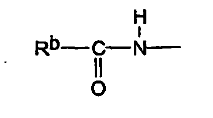 wherein R^{b} is a residue of a peptide or protein. Upon hydrolysis by a peptidase or protease, the M is cleaved from the fluorescence energy transfer label. Upon excitation of the donor moiety at its excitation wavelength, energy is transferred between the donor and acceptor dyes, allowing detection of an increase in fluorescence emission from the acceptor.

In a further embodiment, M comprises a glycosidic linkage and is a substrate for a glycosidase such as α-glycosidases (e.g., α-amylase), β-glycosidases (e.g. β-glucosidase) and has the structure:

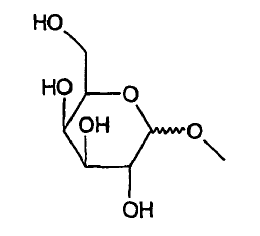

In a further embodiment, M comprises an ether linkage that is a substrate for a dealkylase and having the structure:

R^{c}-O-

wherein R^{c} is C₁ - C₂₀ straight or branched chain alkyl.

In a still further embodiment, group M further comprises a cell membrane permeabilising group, which may be selected from groups such as ether, ester, amide and phospho-diester groups of formula: 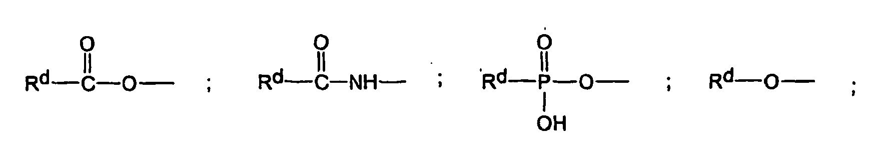 wherein R^{d} is C₁ - C₁₀ straight or branched chain alkyl, either unsubstituted, or substituted with one or more halogen atoms, phenyl, either unsubstituted or substituted with one or more halogen atoms, or a peptide chain. Suitably, halogen atoms may be selected from fluorine, chlorine, bromine and iodine. Useful examples of such membrane permeabilising groups include acetate ester, pivaloyl ester, acetoxymethyl ester, pentafluorobenzyl ether, pentafluorobenzyl amide, pentafluorobenzyl ester, phenyl phosphate, perfluoro-(C₁ - C₆) alkyl ethers, perfluoro-(C₁ - C₆) alkyl esters and perfluoro-(C₁ - C₆) alkyl amide groups. Such groups are substrates for, and are hydrolysed by dealkylases, esterases such as proteases and phospho-diesterases as are found in cells. The skilled person will appreciate the variability of cell permeability of the reporter molecule and will be able to test for the same.

The donor dye is selected from coumarin dyes, benzocoumarin dyes, acridone dyes, xanthine dyes, phenoxazine dyes, rhodamine dyes, merocyanine dyes and cyanine dyes, preferably xanthine dyes and cyanine dyes, wherein the donor dye is capable of transferring energy to the acceptor dye.

The acceptor dye is selected from coumarin dyes, benzocoumarin dyes, acridone dyes, xanthine dyes, phenoxazine dyes, rhodamine dyes, merocyanine dyes and cyanine dyes.

Preferably, the donor dye is a xanthine dye or a cyanine dye and the acceptor dye is a rhodamine or a cyanine dye.

In one preferred embodiment, at least one of said donor and acceptor dye moiety is a cyanine dye. In another preferred embodiment, the donor dye is a xanthine dye and the acceptor dye is a rhodamine dye.

Suitable xanthine dyes include but are not limited to fluorescein and its derivatives, such as 5-carboxyfluorescein, 6-carboxyfluorescein and 6-carboxy-4',5'-dichloro-2',7'-dimethoxyfluorescein.

Suitable cyanine dyes include but are not limited to CyA (3-(ε-carboxypentyl)-3'-ethyl-5,5'-dimethyl oxacarbocyanine), Cy2 (3-(ε-carboxypentyl)-3'-ethyl-oxa-carbocyanine), Cy3 (3-(ε-carboxypentyl)-1'-ethyl-3,3,3',3'-tetramethyl-5,5'-disulphonato-carbocyanine), Cy3.5 (3-(ε-carboxypentyl)-1'-ethyl-3,3,3',3'-tetramethyl-4,5,4',5'-(1,3-disulphonato)dibenzo-carbocyanine), Cy5 (1-(ε-carboxypentyl)-1'-ethyl-3,3,3',3'-tetramethyl-5,5'-disulphonato-dicarbocyanine, Cy5.5 (1-(ε-carboxypentyl)-1'-ethyl-3,3,3',3'-tetramethyl-4,5,4',5'-(1,3-disulphonato)-dibenzo-dicarbocyanine, and Cy7 (1-(ε-carboxypentyl)-1'-ethyl-3,3,3',3'-tetramethyl-5,5'-disulphonato-tricarbocyanine.

Suitable rhodamine acceptor dyes include but are not limited to: 5-carboxyrhodamine (Rhodamine 110-5), 6-carboxyrhodamine (Rhodamine 110-6), 5-carboxyrhodamine-6G (R6G-5 or REG-5), 6-carboxyrhodamine-6G (R6G-6 or REG-6), N,N,N',N'-tetramethyl-5-carboxyrhodamine, N,N,N',N'-tetramethyl-6-carboxyrhodamine (TAMRA or TMR), 5-carboxy-X-rhodamine and 6-carboxy-X-rhodamine (ROX). Other classes of dyes include BODIPY^{™}, porphyrin dyes, rhodol dyes and perylene dyes.

The fluorescent labelled enzyme substrates according to the first aspect may also include water solubilising constituents attached thereto, for conferring a hydrophilic characteristic to the compound. They are preferably attached to aromatic ring systems of the donor or acceptor dye moieties. In the alternative, the linking group L may contain the water solubilising group. Suitable solubilising constituents may be selected from the group consisting of amide, sulphonate, sulphate, phosphate, quaternary ammonium, hydroxyl, guanidinium and phosphonate. Sulphonate or sulphonic acid groups attached directly to the aromatic ring of the donor and/or acceptor fluorochromes are particularly preferred.

Examples of energy transfer reporter molecules are shown in Table 1.

**Table 1 Examples of FRET-coupled Reporter Molecules**

| | |
|---|---|
| 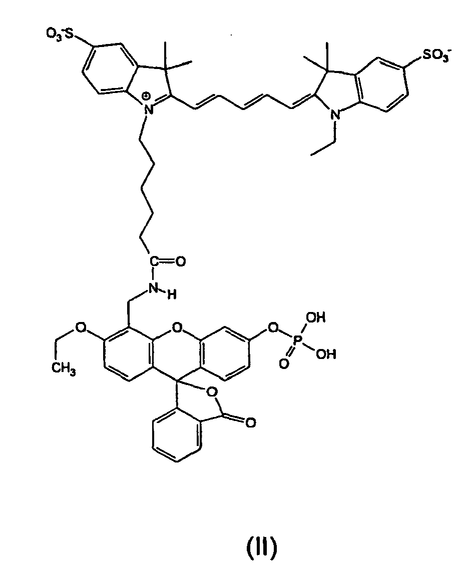 | 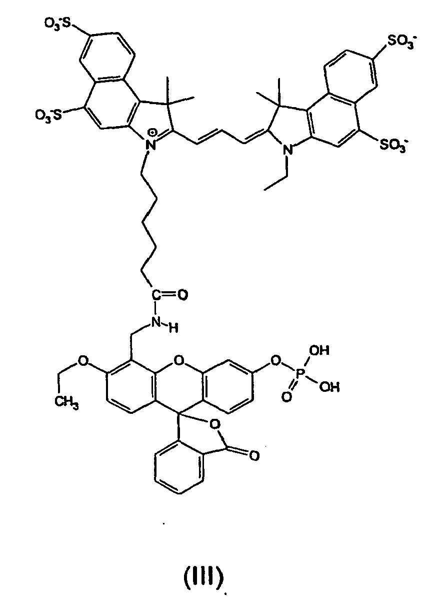 |
| 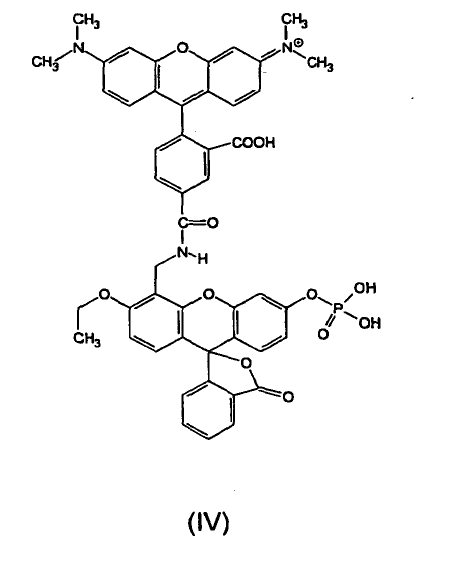 | |

| | |
|---|---|
| 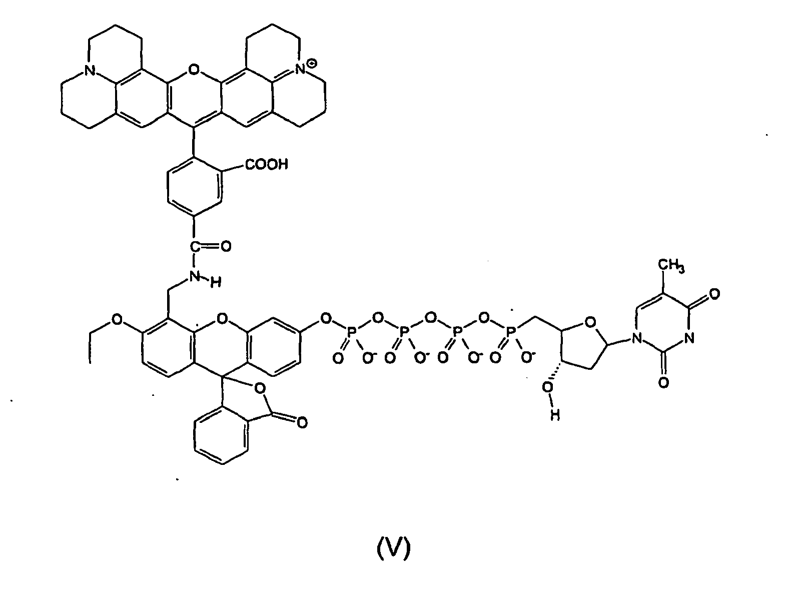 | |
| 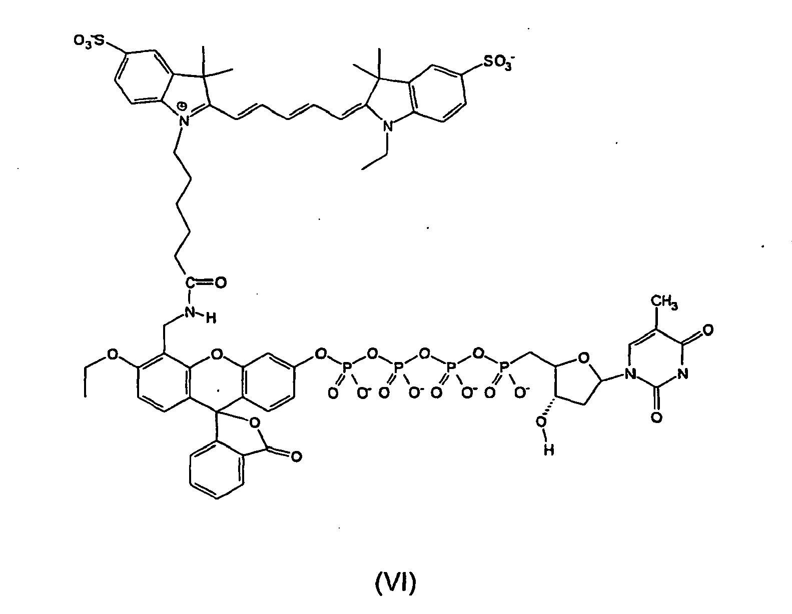 | |
| 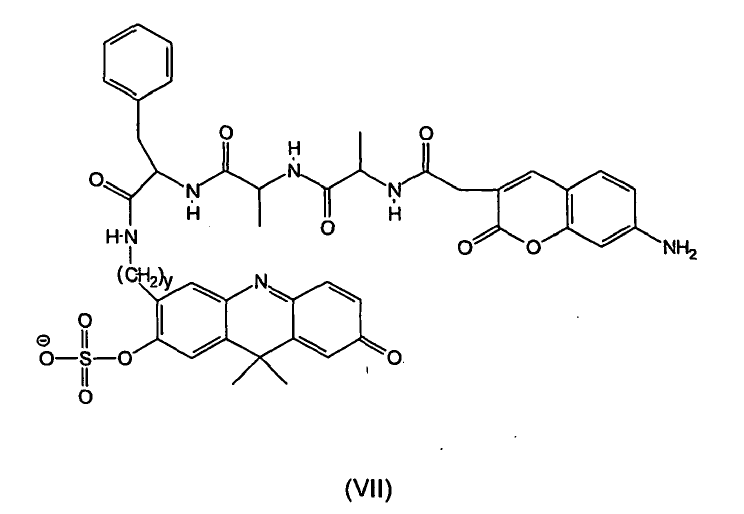 | |

In Examples (II), (III) and (IV), the reporter is a non-fluorescent energy transfer complex containing a quenched fluorescein donor dye and a Cy5, a Cy3 and a rhodamine acceptor dye respectively, the fluorescein moiety having an enzymatically cleavable phosphate group attached thereto. The phosphate group may be cleaved by the action of a phosphatase, thereby restoring fluorescence of the donor dye upon excitation of the fluorescein at its excitation wavelength. Detection of the cleavage event and measurement of phosphatase activity may be achieved by detecting fluorescence emission at the emission wavelength of the acceptor dye.

In Examples (V) and (VI), the reporter is a non-fluorescent energy transfer complex containing a quenched fluorescein donor dye and a Cy5 and rhodamine acceptor dye respectively, the fluorescein moiety having a nucleoside polyphosphate moiety attached thereto. Action of a nucleosidyl phosphotransferase generates a phosphatase substrate *in situ,* which in the presence of phosphatase, undergoes phosphate removal. The fluorescence of the fluorescein donor dye is restored. Subsequent energy transfer to the acceptor dye generates a red-shifted emission. When the nucleosidyl phosphotransferase enzyme is a nucleic acid polymerase, compositions of the present invention can be used for detection, characterization and quantitation of known and unknown nucleic acids.

According to the invention, L is a cleavable linker and includes an enzyme cleavable group P which is different from the enzyme substrate group M. P may be selected from groups such as ether, ester, amide and phospho-diester groups. Such groups are substrates for, and are cleaved in aqueous buffer media by respectively, dealkylases, esterases such as proteases and phospho-diesterases. In this embodiment, it is preferable that D₂ emits at a first emission wavelength λ2 when attached to L, and at a second emission wavelength λ2', once D₂ is cleaved from L, where λ2 ≠ λ2'. The reporter compounds according to the invention are useful for measuring the relative activity of two enzymes in *in vivo* and cellular systems, where it is difficult to ensure equal delivery of two separate enzyme substrates. It is also possible for D₁ to emit at a first emission wavelength (λ1) when attached to M and at a different wavelength (λ1') when M is cleaved, where λ1 ≠ λ1'. Example (VII) shows a dual enzyme reporter for an aryl sulfatase and Cathepsin G activity, that does not form part of the present invention, but which is potentially useful in detecting inflammation. When no enzyme activity is present, there is a weak emission at 610 nm when the compound of Example (VII) is excited at 360 nm. No emission is observed with excitation at 645 nm. With sulfatase activity only, strong emission is observed at 660 nm with excitation at 645 nm and weak emissions at 450 nm and 660 nm when excited at 360 nm. With Cathepsin G activity only, strong emission is observed at 450 nm when excited at 360 nm and no emission at 660 nm upon excitation at either 360 nm or 645 nm. With both enzymes present, strong emission is observed at 450 nm upon excitation at 360 nm and another strong emission at 660 nm upon excitation at 645 nm. No 660 nm emission is observed with excitation at 360 nm. Thus, by measuring emissions at 660 nm and 450 nm and by comparing with known standards it is possible to determine the relative enzyme activities of both enzymes at a particular time point or location in an *in vivo* system. Such dual enzyme activity substrates are useful for distinguishing between different disease states as well as determining the clinical stage or aggressiveness of a disease state.

Both MMP-2 and MMP-9 have been targeted for tumor detection and treatment, for example, in melanomas, expression of these proteins varies significantly. While in very aggressive tumors, MMP-9 is expressed at high levels, no over-expression is observed in early stage tumors. Benign tumors show no MMP-9 activity. Similarly, MMP-2 activity also varies significantly with invasive tumors showing higher activity. Although this is true for the majority of patients, it is not true for all. Therefore, measuring only MMP-2 or MMP-9 activity may lead to misdiagnosis of an aggressive tumor as being benign. For osteoporosis, Cathepsin K has been identified as a therapeutic target as it is over-expressed in osteoclasts; however this activity is also observed during normal bone turnover not associated with osteoporosis. In osteoporosis however, high levels of MMP-9 are also observed. Measuring activity of MMP-9, however, is not a very specific indicator of osteoporosis. Therefore measuring both activities and their ratio provides a better diagnostic tool than a single enzyme activity measurement.

In a second aspect, which does not form part of the present invention, there is provided a method for determining the activity of an enzyme acting on a substrate molecule, the substrate comprising a compound of formula (I), wherein D₁, D₂ and L are hereinbefore defined; and M comprises a substrate for a cleavage enzyme. The method comprises the steps of: i) measuring the fluorescence intensity of the fluorescently labelled substrate; ii) combining an enzyme whose activity is to be determined with the substrate under conditions to cause cleavage of M from D₁; and iii) measuring a change in fluorescence intensity of the fluorescent label following the combination of step ii); wherein the change in fluorescence intensity of the fluorescent label is used to determine the activity of the enzyme.

Suitably, the compound of formula (I) is a substrate for the enzyme whose activity is to be determined, wherein M comprises an enzyme cleavable group preferably selected from a phosphate ester linkage, at least one peptide linkage, an ether linkage and a glycosidic linkage as hereinbefore defined. Preferably, the enzyme is a hydrolase enzyme selected from the group consisting of phosphatase, peptidase, protease, dealkylase and glycosidase.

In one preferred example according to the second aspect, the enzyme is a phosphatase, selected from the E.C. Class 3.1, including PTPase, PPTase-2A, PPTase-2B, or PPTase-2C. Examples are: tyrosine phosphatase, PP1 and PP-2B (serine/threonine phosphatase),

In a second preferred example according to second aspect, the enzyme is a peptidase selected from the E.C. Class 3.4. Examples include, but are not limited to, angiotensin converting enzyme (ACE), caspase, cathepsin D, chymotrypsin, pepsin, subtilisin, proteinase K, elastase, neprilysin, thermolysin, asp-n, matrix metallo proteinase 1 to 20, papain, plasmin, trypsin, enterokinase and urokinase.

In another example, the enzyme is a glycosidase, preferably selected from the group consisting of E.C. Class 3.2, for example α-amylase, β-amylase, glucan 1,4-α-glucosidase, cellulase, endo-1,3-β-glucanase, oligo-1,6-glucosidase and lysozyme.

In a further example, the enzyme is a dealkylase, for example different isozymes of cytochrome P-450.

Other enzymes, such as peroxidases, β-lactamase, nitroreductase, etc. may also be used with appropriate M or linker structures. Fluorogenic substrates for these enzymes are well known, e.g. Amplex red, a fluorescein and 6-chloro-7-hydroxy coumarin substituted cephalosporin, and nitrocyanines, such as those described in (EP 1086179 B1, Hamilton, A. et al). Similar structures may be incorporated in the compositions described above.

Assays may be performed according to the present invention in high throughput screening applications, including those in which test agents are screened for their inhibitory effects, potentiation effects, agonistic, or antagonistic effects on the enzyme reaction under investigation. Thus, in a preferred example according to the second aspect, there is provided a method of screening for a test agent whose effect upon the activity of an enzyme in cleaving a substrate is to be determined. The method comprises the steps of: (a) performing the method according to the second aspect in the presence and in the absence of the test agent; and (b) determining the activity of the enzyme in the presence and in the absence of the agent; wherein a difference between the activity of the enzyme in the presence and in the absence of said agent is indicative of the effect of the test agent on the activity of the enzyme. Alternatively, the screening can be carried out by performing the method in the presence of a test agent and comparing the value of the activity of the enzyme with a control value for the enzyme activity in the absence of the test agent. The control value may be conveniently stored electronically in a database or other electronic format.

In a third aspect of the invention, there is provided a method of determining relative activities of two enzymes acting on a substrate molecule, the substrate molecule comprising a compound of formula (I), wherein D₁, D₂, M and L are as defined in the first aspect of the invention. The method comprises the steps of: i) measuring the fluorescence emission intensity of the fluorescently labelled substrate; ii) combining the first and second enzymes with the substrate under conditions to cause cleavage of M from D₁ and D₂ from D₁; iii) measuring the fluorescence emission intensities of the first and second dye moieties following the combination of step ii); and iv) utilising a change in fluorescence intensities of the first and second dye moieties to determine the relative activities of the enzymes.

Suitably, the first and second enzymes may be combined either sequentially, in either order or, alternatively, simultaneously with the substrate molecule. Preferably, the first and second enzymes are selected from the group consisting of phosphatase, peptidase, protease, dealkylase and glycosidase, as hereinbefore described.

The method according to the third aspect of the invention may also be used to determine the effect of a test agent on the relative activities of two enzymes acting on a substrate molecule, in which the combining step ii) is performed in the absence and in the presence of the test agent. A difference between the relative activities of the first and second enzymes in the presence and in the absence of said agent is indicative of the effect of the test agent on the relative activities of the first and second enzymes.

The test agent may be, for example, any organic or inorganic compound such as a synthetic molecule or a natural product (e.g. peptide, oligonucleotide), or a may be an energy form (e.g. light or heat or other forms of electromagnetic radiation). Inhibitors of enzyme activity can be detected using the method according to the preferred embodiment; for example, the known inhibitor pepstatin A can be readily shown to inhibit cathepsin D enzyme activity using the method of the invention. Thus, in the presence of 12 nM of pepstatin A, cathepsin D activity is some 5 fold less than in the absence of the inhibitor.

Suitably, the difference between the activity of the enzyme in the absence and in the presence of the agent is normalised, stored electronically and compared with a reference value. Thus, for example, the difference in activity may be stored as a percentage inhibition (or percentage stimulation) on an electronic database and this value compared with the corresponding value for a standard inhibitor of the enzyme in question. In this way, only test agents meeting a certain pre-determined threshold (e.g. as being as effective or more effective than the reference compound) may be selected as being of interest for further testing.

As an example, an assay for the detection of proteolytic enzyme activity may be configured as follows. A reaction mixture is prepared by combining an enzyme and a fluorescent energy transfer reporter compound according to formula (I), wherein group M comprises a proteolytic enzyme cleavage site. The assay is suitably performed in the wells of a multiwell plate, e.g. a microtitre plate having 24, 96, 384 or higher densities of wells, e.g. 1536 wells. The reaction may be performed with the enzyme substrate initially present in an aqueous assay buffer, suitably, 10mM MOPs, 50mM Tris or 50mM HEPES, containing 5 mM MgCl₂. A test agent, such as a known or a putative inhibitor, may be optionally included in the reaction mixture. Upon addition of enzyme, typically the reaction is allowed to proceed to completion, progress being monitored by observing the steady state fluorescence emission due to the fluorescent acceptor dye, which is recorded using a spectrofluorimeter. Alternatively, the assay may be performed under "stopped" conditions in which the reaction is allowed to proceed for a predetermined time and then terminated with a stop reagent, normally an inhibitor of the enzyme activity, which is often non-specific. An example of a stop reagent is EDTA, which is used to sequester metal ions that are normally required for enzymatic activity.

The methods according to the present invention may also be employed to measure the activity of an enzyme acting on a substrate in a cellular environment, the substrate comprising a compound according to the present invention. Thus in a particular example and embodiment according to the second and third aspects, respectively, the method comprises, before step i) the step of adding the substrate to one or more cells in a fluid medium.

Typically, cultured cells are incubated with the FRET-coupled enzyme substrate at a concentration of 0.1 to 100µM in a suitable cell culture medium under conditions suitable for cell growth and for a time which may range from 0.5 to 24 hours. Cells are cultured according to standard cell culture techniques, e.g. cells are cultured in a suitable vessel in a sterile environment at 37°C in an incubator containing a humidified 95% air/5% CO₂ atmosphere. There are established protocols available for the culture of diverse cell types. (See for example, Freshney, R.I., Culture of Animal Cells: A Manual of Basic Technique, 2nd Edition, Alan R.Liss Inc. 1987). When the substrate is required to be introduced into cells grown in cell or tissue culture, the substrate is simply added to the culture medium. Cells may also be contacted with the substrate in the presence of a test agent whose effect on the enzyme activity is to be determined. In this embodiment, the detection step provides a measurement of the effect of the test agent on the activity of the enzyme under investigation.

Measurements of fluorescence intensity may be made using instruments incorporating photo-multiplier tubes as detectors. Changes in fluorescence intensity may be measured by means of a charge coupled device (CCD) imager (such as a scanning imager or an area imager) to image all of the wells of a microtitre plate. The LEADseeker^{™} system features a CCD camera allowing fluorescence imaging of high density microtitre plates in a single pass. Imaging is quantitative and fast, and instrumentation suitable for imaging applications can now simultaneously image the whole of a multiwell plate. Where an assay is to be formatted for the determination of the activity of a test agent on enzyme activity, the assay may be performed under continuous measurement of the fluorescence of the substrate. In this format, the intensity of the fluorescent labelled substrate changes continuously. The labelled substrate does not need separation from the product of the enzymatic reaction and thus, a time-course of the reaction may be obtained, allowing kinetic studies to be performed in real time.

In a fourth aspect of the present invention, there is provided a test kit for measuring the activity of at least one enzyme, the test kit comprising one or more different enzyme substrates, each of said substrates comprising a compound of formula (I), wherein D₁, D₂, L and M are hereinbefore defined. Preferably, the test kit further comprises at least one or more different enzymes each enzyme specific for a different said substrate.

The compounds of formula (I) may be prepared by covalent attachment of the fluorescence energy transfer moiety, D₁ - L - D₂, to group M wherein D₁, D₂, M and L are hereinbefore defined, using direct chemical coupling methods that are well known to the skilled person. Alternatively, the compounds of the present invention may be prepared by *de novo* synthetic methods as illustrated in the examples. Group M may be initially attached to D₁ to form the intermediate compound, M-D₁, followed by attachment of D₂ via linker group L. The linker group L may be conveniently attached to M-D₁ prior to covalent attachment of D₂, or alternatively L may be pre-attached to D₂. Peptide, protein and oligonucleotide substrates for use in the invention may be labelled at a terminal position, or alternatively at one or more internal positions. For reviews and examples of protein labelling using fluorescent dye labelling reagents, see "Non-Radioactive Labelling, a Practical Introduction", Garman, A.J. Academic Press,1997; "Bioconjugation - Protein Coupling Techniques for the Biomedical Sciences", Aslam, M. and Dent, A., Macmillan Reference Ltd, (1998). Protocols are available to obtain site specific labelling in a synthesised peptide, for example, see Hermanson, G.T., Bioconjugate Techniques, Academic Press (1996).

In order to clarify the principle and the function of the invention, reference is now made to the following examples.

### Examples

### 1. Preparation of FRET-coupled Reporter Molecules (Compound (V) and Compound (VI) (See Figure 2)

### 1.1 4'(5')-Fluorescein diethylether-ester: Compound (2)

To a solution of NaOH (1.44 g) in methanol (145 ml) was added fluorescein (6.0 g) to give a dark colored solution which was concentrated to dryness *in vacuo.* This was co-evaporated with anhydrous DMF (2x50 ml) and redissolved in anhydrous DMF (150 ml). Ethyl iodide (11.6 ml, 8 eq) was added and reaction stirred at room temperature for overnight. After concentration of the reaction mixture, it was dissolved in saturated sodium bicarbonate solution (150 ml) and washed with diethylether. A yellow precipitate formed on cooling of the solution which was filtered and finally washed with ether (2x 25 ml) and ether/pentane (1:1, 50 ml).

### 1.2 4'(5')-Chloroacetylaminomethyl-3'(6')-ethylfluorescein diethylether-ester: Compound (3)

To a stirred solution of ethylfluorescein ester (Compound (2), 2.67 g) in 50 ml sulfuric acid, chloroacetylacetamide (850 mg) was added slowly over 5 minutes. The reaction mixture was stirred at room temperature overnight. The reaction mixture was poured over crushed ice and the precipitate formed was filtered and washed with cold water. The TLC and mass analysis indicated the formation of mono and bis-chloroacetamido compounds. The products were purified by silica gel column chromatography using 2-15% methanol in methylene chloride. The bis-alkylated product was eluted first followed by two mono-alkylated products. The lower mono-alkylated product was the major product, which was used for further reactions.

### 1.3 4'(5')-Aminomethyl-3'(6')-ethylfluorescein: Compound (5)

Major monoalkylated product (Compound (3), 1g) was hydrolyzed with 6N HCl (15 ml) by heating for 2.5 hr. The product was extracted with CH₂Cl₂, washed with water and after drying over anhydrous sodium sulfate, purified by silica gel column chromatography to give 500 mg pure compound. MS ES+: 390.3

### 1.4 4'(5)-Trifluoroacetylamidomethyl-3'(6')-ethylfluorescein: Compound (6)

Compound (5) (390 mg) was taken up in anhydrous pyridine (5 ml) and TFA-NHS (650 mg) was added. The mixture was stirred overnight at room temperature. After concentration *in vacuo* and extraction with methylene chloride, the product was purified by silica gel chromatography to give 350 mg. pure product. MS ES+: 486.3

### 1.5. 4'(5')-Trifluoroacetylamidomethyl-3'(6')-ethylfluorescein-6'(3')-phosphate: Compound (7)

To a solution of Compound (6) (200 mg) in acetonitrile (5 ml), POCl₃ (189 mg, 3 eq) was added and the reaction mixture stirred at room temperature for 1 hour. Pyridine (290 µl, 9 eq) was added and the reaction stirred for a further 2 hr. The reaction was quenched by adding TEAB (0.1 M, 10 ml). After 1 hr, the mixture was concentrated *in vacuo* and purified by silica gel chromatography using CH₂Cl₂/methanol gradient to give 130 mg pure monophosphate. MS ES⁻: 564.5

### 1.6 4'(5')-Aminomethyl-3'(6')-ethylfluorescein-6'(3')- tetraphosphate-thymidine: Compound (8)

### 1.6.1 Synthesis of the imidazolide derivative of Compound (7)

To a solution of Compound (7) (60 mg) in anhydrous DMF (2 ml) was added tributylamine (30 µl, 3 eq), and the mixture was concentrated to dryness *in vacuo.* The mixture was redissolved in anhydrous DMF (2 ml) and carbonyldiimidazole (85 mg, 5 eq) was added with stirring. The mixture was stirred at room temperature for 2 hr and checked for completion by HPLC-MS. The mixture was quenched with methanol (100 µl), stirred at room temperature for 1 hr, concentrated to dryness *in vacuo* and redissolved in anhydrous DMF (2 ml). MS ES⁻: 614.6

### 1.6.2 Addition of imidazolide derivative of Compound (7) to thymidine-5'-triphosphate

Thymidine-5'-triphosphate (triethylammonium salt, 100 µmol) was co-evaporated with tributylamine (400 µmol) in anhydrous DMF (2x2 ml) and finally redissolved in anhydrous DMF (2 ml).

To the above tributylammonium salt solution of thymidine-5'-triphosphate (TTP), imidazolide derivative (from step 1.6.1) was added and the mixture stirred at room temperature for overnight. HPLC-MS indicated >90% complete reaction. This was further stirred for another 5 hr.

### 1.6.3 The reaction mixture from step 1.6.2 was concentrated and NH₄OH (30% solution, 10 ml) was added

The mixture was stirred at room temperature for 2.5 hr and left in a refrigerator overnight. The mixture was concentrated to dryness *in vacuo* and purified by anion exchange chromatography. This mixture was treated with snake venom phosphodiesterase (SVD) and alkaline phosphatase overnight (to remove any unreacted TTP) and purified further by reverse phase chromatography using 0.1 M TEAB (buffer A) to 25% acetonitrile in 0.1 M TEAB. Two major peaks were collected and analyzed by HPLC-MS. The first major peak gave mass at 931.8 and the second peak gave mass at required 932.8.

It was apparent that that the ammonia treatment during the hydrolysis of trifluoroacetyl group also reacted with the spirolactone and resulted in the formation of undesired amido compound. Peak 2 was concentrated to dryness *in vacuo,* co-evaporated with methanol (2x5 ml) and redissolved in H₂O (1ml). The concentration was measured by UV. A total of 5.5 µmol pure compound was obtained.

### 1.7 Addition of Acceptor Dyes to Compound (8): Synthesis of Compounds (V) and (VI)

To two separate reaction vessels of Compound (8) (2.2 µmol, 400 µl of 5.5 mM solution) was added 400 µl of 0.5 M sodium carbonate/sodium bicarbonate buffer (pH 8.5) and in one vessel 5-ROX-NHS ester (5.5 mg in 2 ml DMF) and in another vessel Cy5-NHS ester (5 mg in 2 ml DMF) was added. Both reaction mixtures were stirred at room temperature for overnight and monitored by HPLC-MS for the completion of the reaction. The reaction mixtures were concentrated *in vacuo* and purified first on ion exchange column using 25% in acetonitrile in 0.1 M TEAB (buffer A) and 25% acetonitrile in 1 M TEAB (buffer B). The peak containing the product was concentrated *in vacuo* and further repurified by reverse phase HPLC column. The correct fractions were collected and concentrated *in vacuo* and quantified by UV spectroscopy. Compound (V): MS ES⁻: 1449;
Compound (VI): MS ES⁻: 1572

### 2. Synthesis of Compounds (II), (III) and (IV)

The synthesis was carried out as in the manner described reported above for Compounds (V) and (VI), except that the starting material was 4'(5')-aminomethyl-ethoxyfluorescein phosphate (obtained by hydrolysis of Compound (7) with K₂CO₃ in methanol). The products were purified by HPLC and quantified by UV spectroscopy.
Compound (II): MS ES⁻: 1107.3;
Compound (III): MS ES⁻: 1342.4;
Compound (IV): MS ES⁻: 881.

### 3. Action of Alkaline Phosphatase on Compounds (II), (III) and (IV)

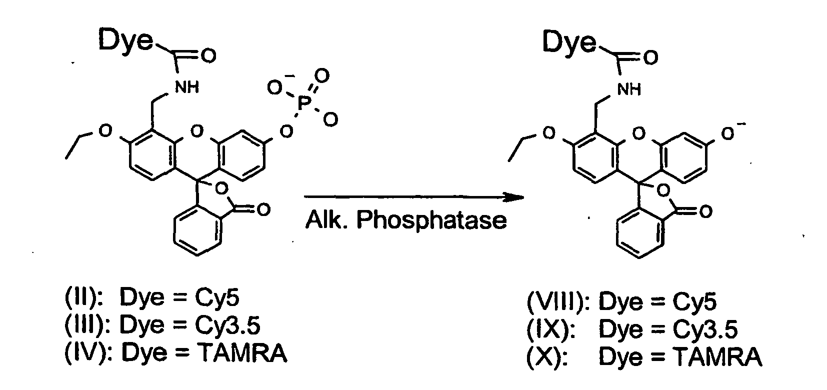

Compounds II, III and IV were taken up in 25 mM HEPES and 5 mM MgCl₂ buffer (pH 8.5) and treated with alkaline phosphates for 2 hours at room temperature. The reaction was followed spectroscopically, which indicated that the reaction was essentially complete within 30 minutes.

### 4. UV, Fluorescence and Energy Transfer Measurements on Compounds (II), (III), (IV), (VIII), (IX) and (X)

The UV and fluorescence spectra of phosphorylated (Compounds (II), (III) and (IV)) and non-phosphorylated dyes (Compounds (VIII), (IX) and (X)) are shown in Figures 2-8. UV spectra clearly show the appearance of peak(s) at about 472 nm upon treatment with alkaline phosphatase.

Having described the particular, desired embodiments of the invention herein, it should be appreciated that modifications may be made as long as they do not depart from the true scope of the invention as set forth in the claims appended hereto.

## Claims

1. A compound of formula (I):
M—D₁—L—D₂ (I)
wherein:
D₁ is a first dye moiety whose fluorescence properties may be modulated so as to be suitable as a donor in an energy transfer arrangement, wherein D₁ is capable of transferring energy to D₂ and is selected from: coumarin dyes, benzocoumarin dyes, acridone dyes, xanthine dyes, phenoxazine dyes, rhodamine dyes, merocyanine dyes and cyanine dyes, preferably xanthine dyes and cyanine dyes;
D₂ is a second dye moiety suitable as an acceptor in an energy transfer arrangement with said first dye, and is selected from: coumarin dyes, benzocoumarin dyes, acridone dyes, xanthine dyes, phenoxazine dyes, rhodamine dyes, merocyanine dyes and cyanine dyes;
M is an enzyme cleavable group chosen to modulate the fluorescence properties of D₁, wherein M comprises a substrate for a first cleavage enzyme;
L is a linking group comprising 2-200 linked atoms, wherein said linking group is a cleavable linker and includes an enzyme cleavable group P which is a substrate for a second cleavage enzyme different from said first cleavage enzyme.

2. A compound according to claim 1, wherein said first cleavage enzyme is selected from the group consisting of a peptidase, a protease, a phosphatase, a dealkylase and a glycosidase.

3. A compound according to either of claims 1 or 2, wherein M is the group: 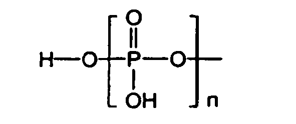 wherein n is an integer from 1 to 4.

4. A compound according to either of claims 1 or 2, wherein M comprises at least one peptide linkage (-CO-NH-) covalently bonded to D₁.

5. A compound according to either of claims 1 or 2, wherein M comprises a glycosidic linkage that is a substrate for a glycosidase.

6. A compound according to either of claims 1 or 2, wherein M comprises an ether linkage that is a substrate for a dealkylase.

7. A compound according to either of claims 1 or 2, wherein M further comprises a cell membrane permeabilising group.

8. A compound according to claim 7, wherein said cell membrane permeabilising group is selected from groups: 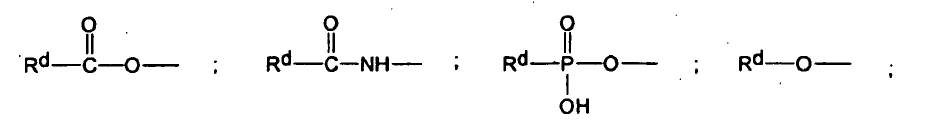 wherein R^{d} is:
C₁ - C₁₀ straight or branched chain alkyl, either unsubstituted, or substituted with one or more halogen atoms;
phenyl, either unsubstituted or substituted with one or more halogen atoms; or
a peptide chain.

9. A compound according to any one of claims 1 to 8, wherein linking group L is selected from carbon atoms which include one or more groups selected from -C(O)-, -NR'-, -O-, -S-, -CH=CH-, -CO-NH-, phenylenyl and the group: 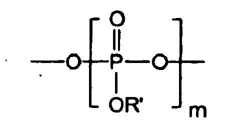 where R' is selected from hydrogen and C₁ - C₄ alkyl and m is an integer from 1 to 3.

10. A compound according to any one of claims 1 to 8, wherein linking group L comprises a peptide or an oligonucleotide fragment.

11. A compound according to any one of claims 1 to 10, wherein said donor dye is a xanthine dye or a cyanine dye and the acceptor dye is a rhodamine or a cyanine dye.

12. A compound according to any one of claims 1 to 10, wherein at least one of said donor and acceptor dye moiety is a cyanine dye.

13. A compound according to any one of claims 1 to 10, wherein said donor dye is a xanthine dye and said acceptor dye is a rhodamine dye.

14. A compound according to any of the previous claims further comprising water solubilizing constituents attached thereto, said water solubilizing constituents being selected from the group consisting of sulphonate, sulphonic acid, phosphate, phosphonate, quaternary ammonium and hydroxyl.

15. A method of determining relative activities of two enzymes acting on a substrate molecule, said substrate molecule comprising a compound of formula I:
M D₁-L-D₂
wherein D₁, D₂, M and L areas defined in Claim 1;
the method comprising the steps of:
i) measuring the fluorescence emission intensity of the fluorescently labelled substrate;
ii) combining said first and second enzymes with said substrate under conditions to cause cleavage of M from D₁ and D₂ from D₁;
iii) measuring the fluorescence emission intensities of said first and second dye moieties following the combination of step ii): and
iv) utilising a change in fluorescence intensities of said first and second dye moieties to determine the relative activities of said enzymes.

16. A method according to claim 15 wherein said first and said second enzymes are selected from the group consisting of phosphatase, peptidase, protease, dealkylase and glycosidase.

17. A method according to claim 15 or claim 16 wherein said combining step ii) is performed in the absence and in the presence of a test agent whose effect on the relative activities of said first and second enzymes is to be determined; wherein a difference between the relative activities of said first and second enzymes in the presence and in the absence of said agent is indicative of the effect of said test agent on the relative activities of said first and second enzymes.

18. A test kit for determining the activity of an enzyme, said kit comprising one or more different enzyme substrates, each of said substrates comprising a compound of formula (I) as defined as in claim 1, wherein D₁, D₂, L and M are defined as in claim 1.

19. A test kit according to claim 18 further comprising one or more different said enzymes each enzyme specific for a different said substrate.

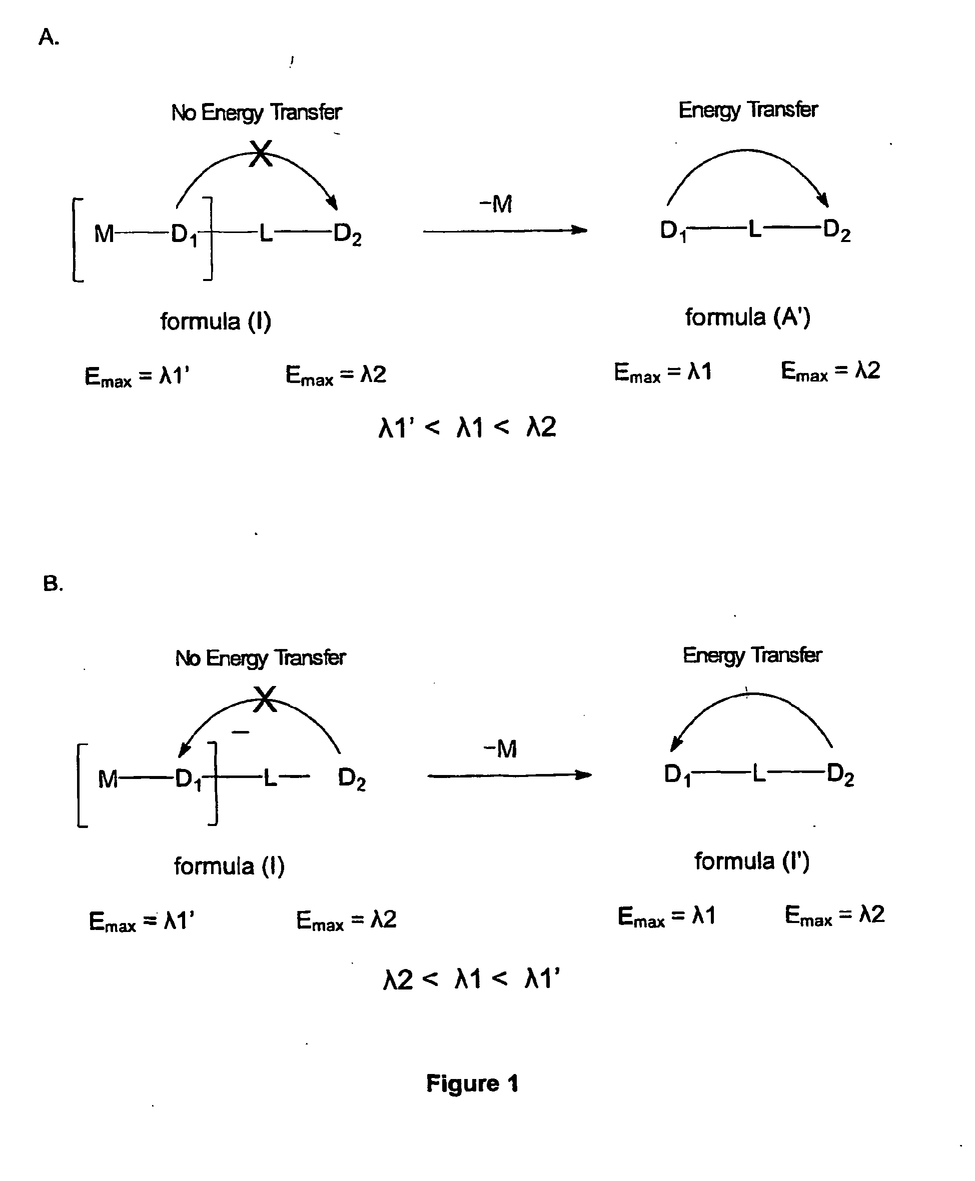

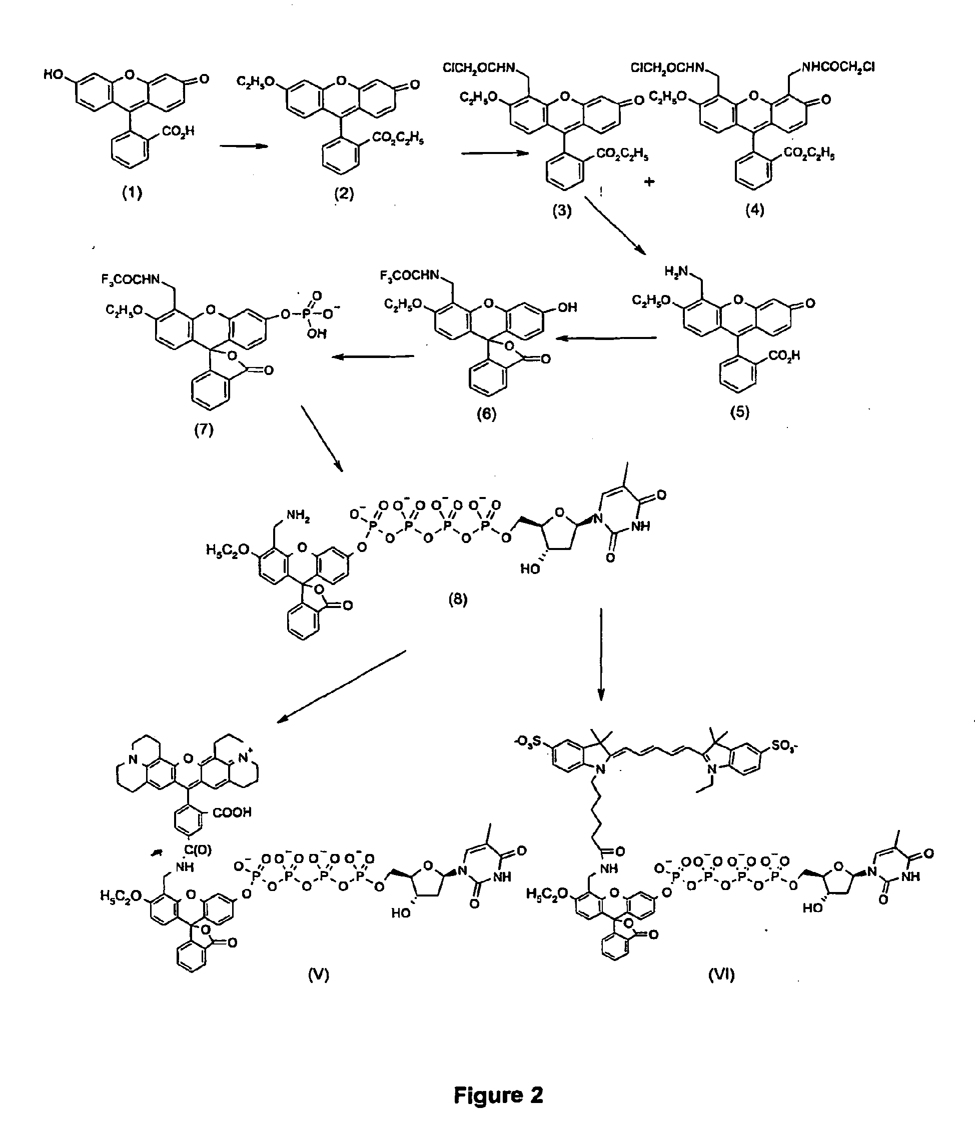

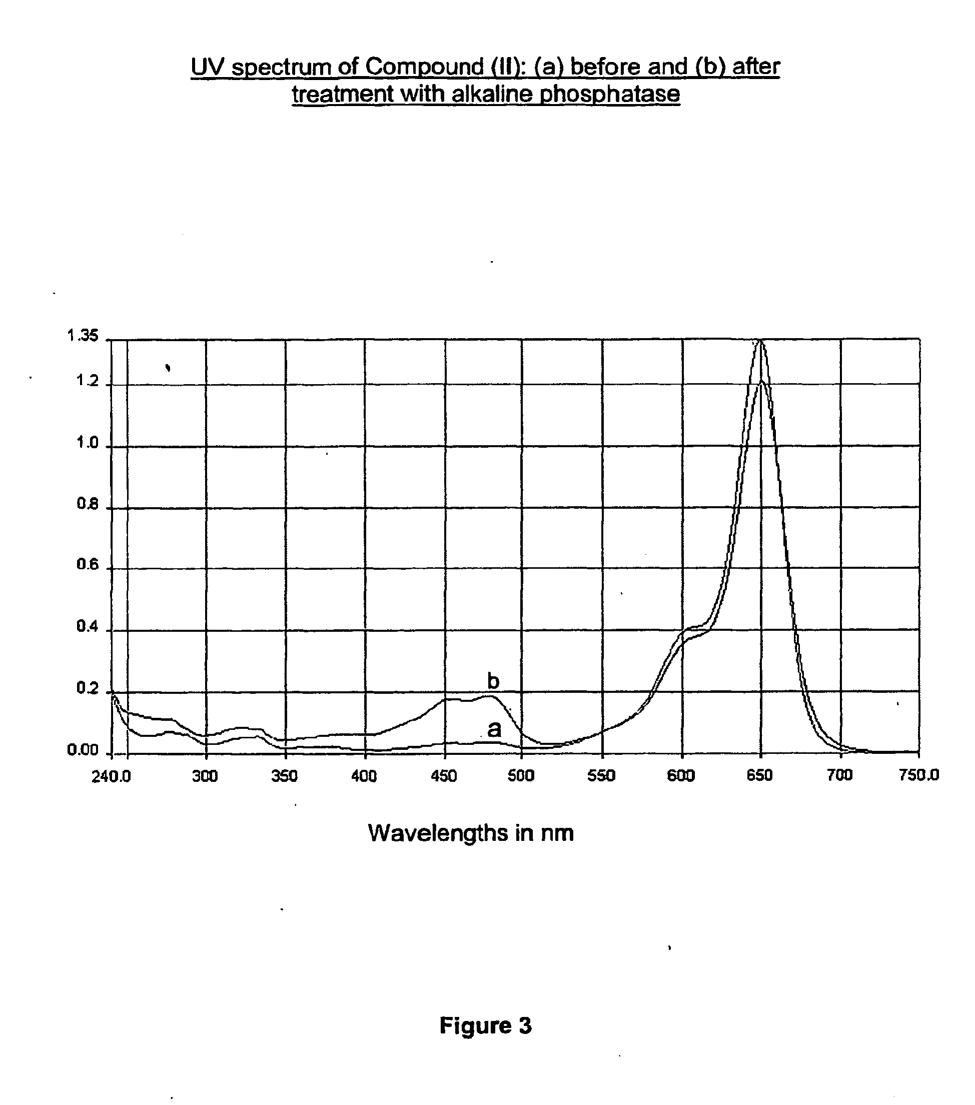

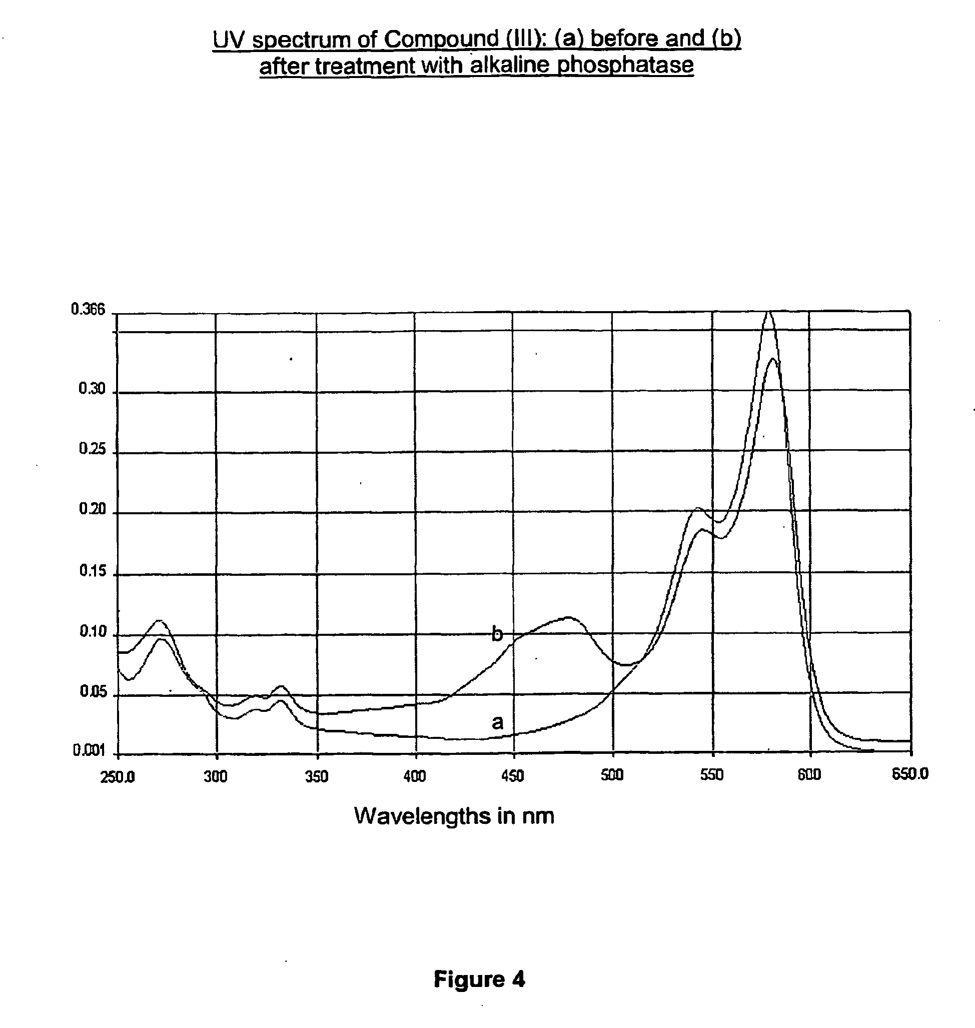

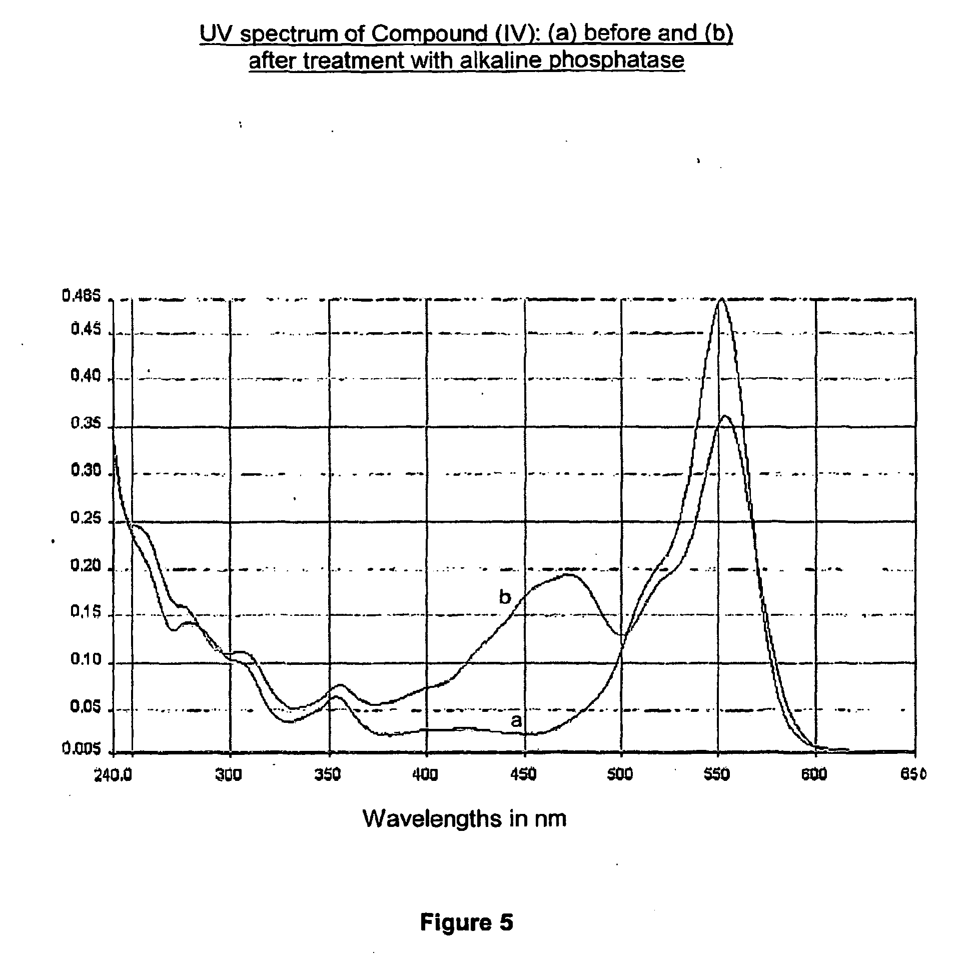

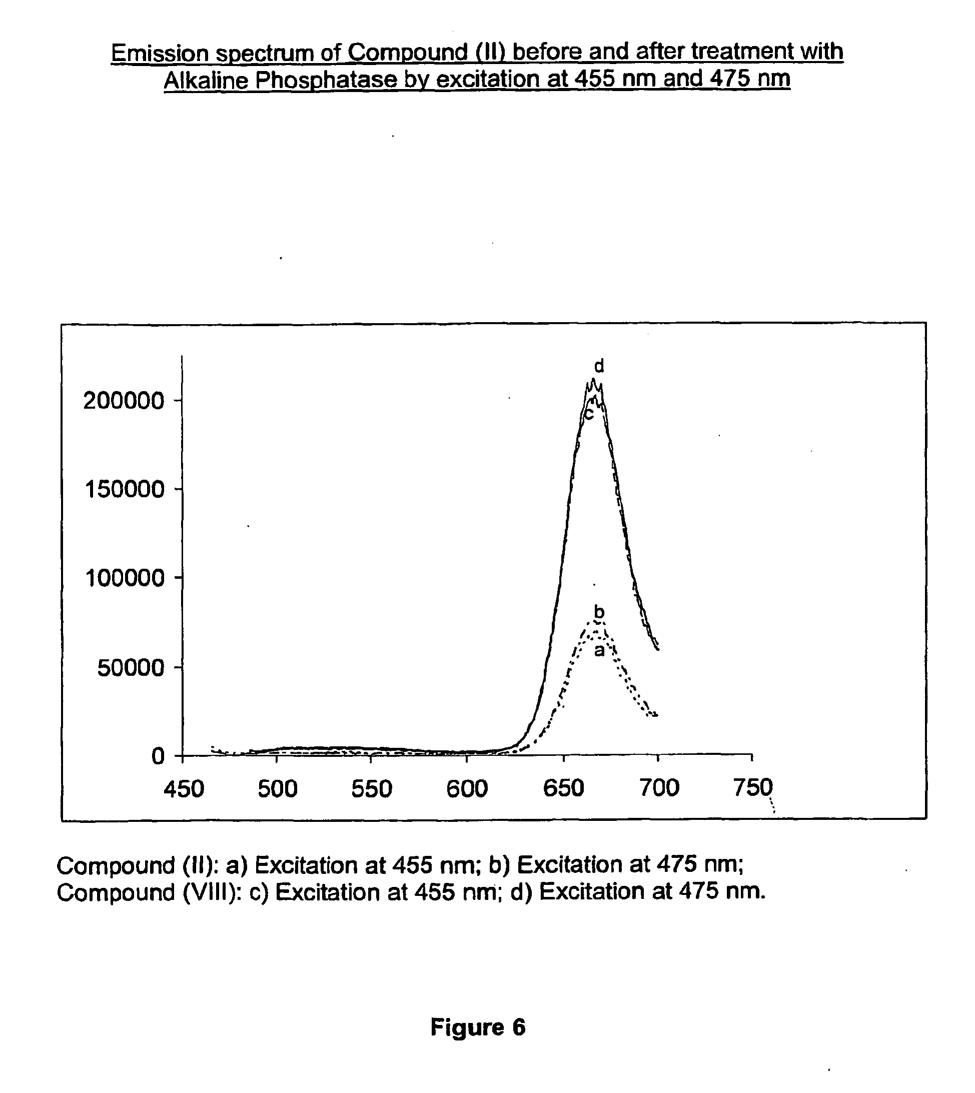

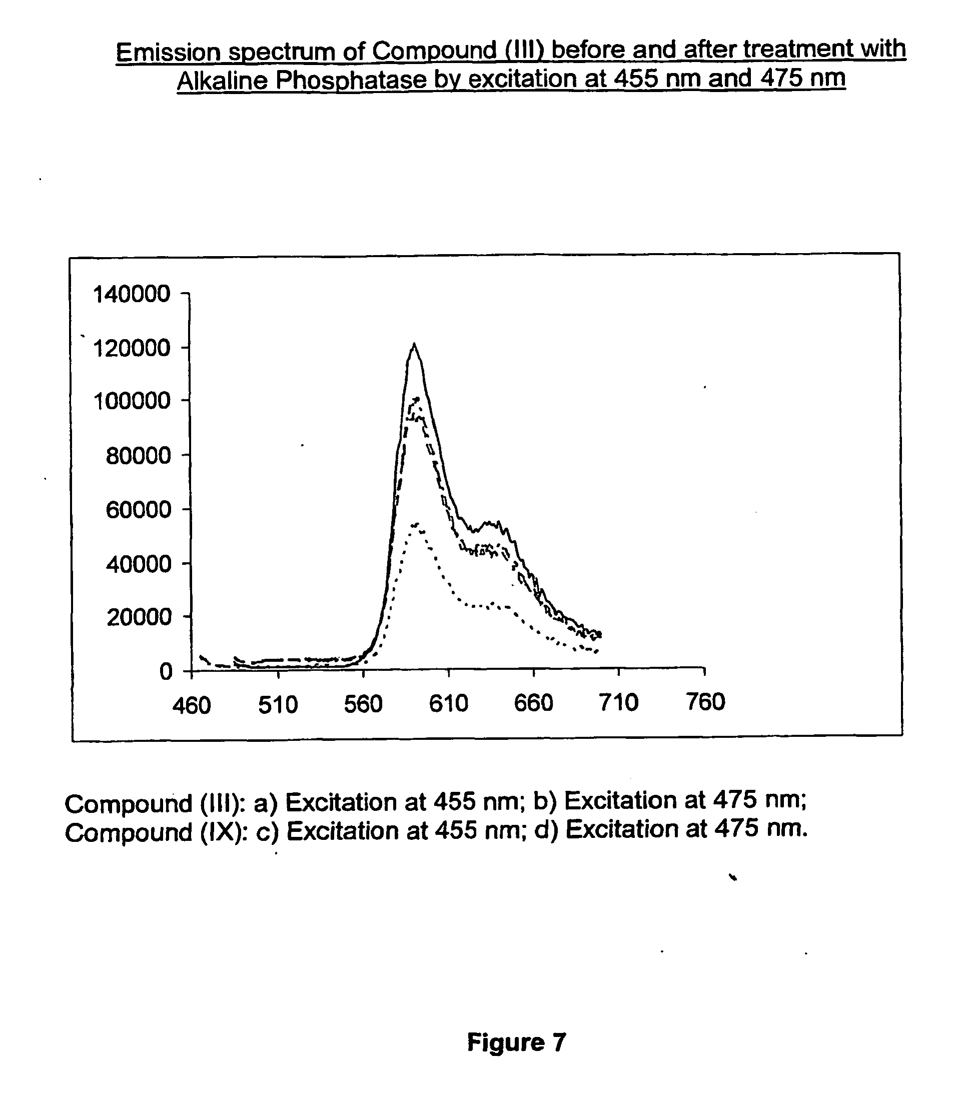

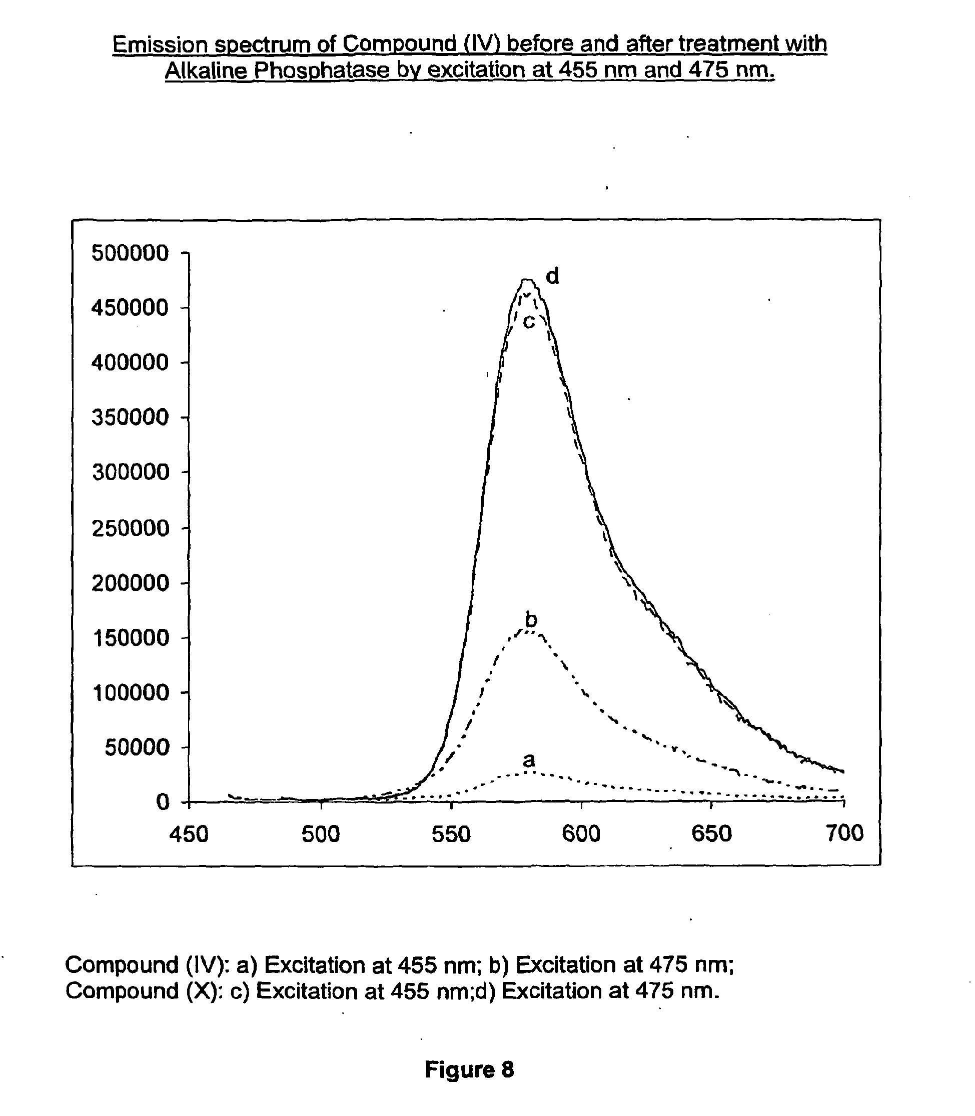

## Patentansprüche

1. Verbindung der Formel (I):
M-D1-L-D₂ (I)
wobei:
D₁ ein erster Farbstoffteil ist, dessen Fluoreszenzeigenschaften so moduliert werden können, dass er sich als Donator in einer Energietransferanordnung eignet, wobei D₁ in der Lage ist, Energie an D₂ zu übertragen, und ausgewählt ist aus: Cumarinfarbstoffen, Benzocumarinfarbstoffen, Acridonfarbstoffen, Xanthinfarbstoffen, Phenoxazinfarbstoffen, Rhodaminfarbstoffen, Merocyaninfarbstoffen und Cyaninfarbstoffen, bevorzugt Xanthinfarbstoffen und Cyaninfarbstoffen;
D₂ ein zweiter Farbstoffteil ist, der sich als Akzeptor in einer Energietransferanordnung mit dem ersten Farbstoff eignet, und ausgewählt ist aus: Cumarinfarbstoffen, Benzocumarinfarbstoffen, Acridonfarbstoffen, Xanthinfarbstoffen, Phenoxazinfarbstoffen, Rhodaminfarbstoffen, Merocyaninfarbstoffen und Cyaninfarbstoffen;
M eine enzymspaltbare Gruppe ist, ausgewählt, um die Fluoreszenzeigenschaften von D₁ zu modulieren, wobei M ein Substrat für ein erstes Spaltungsenzym umfasst;
L eine Verbindungsgruppe ist, die 2 - 200 verbundene Atome umfasst, wobei die Verbindungsgruppe ein spaltbarer Linker ist und eine enzymspaltbare Gruppe P beinhaltet, die ein Substrat für ein zweites Spaltungsenzym darstellt, das sich vom ersten Spaltungsenzym unterscheidet.

2. Verbindung nach Anspruch 1, wobei das erste Spaltungsenzym aus jener Gruppe ausgewählt ist, die aus einer Peptidase, einer Protease, einer Phosphatase, einer Dealkylase und einer Glykosidase besteht.

3. Verbindung nach Anspruch 1 oder 2, wobei M die Gruppe ist: 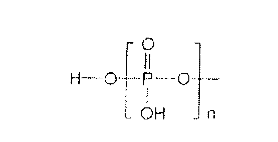 wobei n eine ganze Zahl von 1 bis 4 ist.

4. Verbindung nach Anspruch 1 oder 2, wobei M zumindest eine Peptidbindung (-CO-NH-) umfasst, die kovalent an D₁ gebunden ist.

5. Verbindung nach Anspruch 1 oder 2, wobei M eine glykosidische Bindung umfasst, die ein Substrat für eine Glykosidase ist.

6. Verbindung nach Anspruch 1 oder 2, wobei M eine Etherbindung umfasst, die ein Substrat für eine Dealkylase ist.

7. Verbindung nach Anspruch 1 oder 2, wobei M weiterhin eine Zellmembran-Permeabilisierungsgruppe umfasst.

8. Verbindung nach Anspruch 7, wobei die Zellmembran-Permeabilisierungsgruppe ausgewählt ist aus den Gruppen: 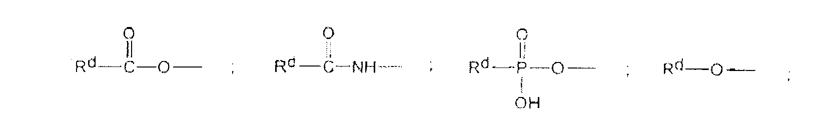 wobei R^{d} ist:
gerad- oder verzweigtkettiges C₁-C₁₀-Alkyl, entweder unsubstituiert, oder substituiert mit einem oder mehreren Halogenatomen;
Phenyl, entweder unsubstituiert oder substituiert mit einem oder mehreren Halogenatomen; oder
eine Peptidkette.

9. Verbindung nach einem der Ansprüche 1 bis 8, wobei Verbindungsgruppe L aus Kohlenstoffatomen ausgewählt ist, die eine oder mehrere Gruppen beinhalten, ausgewählt aus -C(O)-, -NR'-, -O-, -S-, -CH=CH-, -CO-NH-, Phenylenyl und der Gruppe: 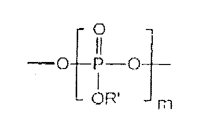 worin R' aus Wasserstoff und C₁-C₄-Alkyl ausgewählt ist und m eine ganze Zahl von 1 bis 3 ist.

10. Verbindung nach einem der Ansprüche 1 bis 8, wobei Verbindungsgruppe L ein Peptid- oder ein Oligonukleotidfragment umfasst.

11. Verbindung nach einem der Ansprüche 1 bis 10, wobei der Donatorfarbstoff ein Xanthinfarbstoff oder ein Cyaninfarbstoff ist und der Akzeptorfarbstoff ein Rhodamin- oder ein Cyaninfarbstoff ist.

12. Verbindung nach einem der Ansprüche 1 bis 10, wobei zumindest einer vom Donator- und Akzeptorfarbstoffteil ein Cyaninfarbstoff ist.

13. Verbindung nach einem der Ansprüche 1 bis 10, wobei der Donatorfarbstoff ein Xanthinfarbstoff ist und der Akzeptorfarbstoff ein Rhodaminfarbstoff ist.

14. Verbindung nach einem der vorstehenden Ansprüche, weiterhin umfassend daran angebrachte wasserlöslich machende Bestandteile, wobei die wasserlöslich machenden Bestandteile aus jener Gruppe ausgewählt sind, die aus Sulfonat, Sulfonsäure, Phosphat, Phosphonat, quaternärem Ammonium und Hydroxyl besteht.

15. Verfahren zur Feststellung relativer Aktivitäten zweier Enzyme, die auf ein Substratmolekül wirken, wobei das Substratmolekül eine Verbindung der Formel umfasst:
M - D₁-L -D₂
wobei D₁, D₂, M und L sind, wie in Anspruch 1 definiert;
wobei das Verfahren die Schritte umfasst:
i) Messen der Fluoreszenzemissionsintensität des fluoreszierend markierten Substrats;
ii) Kombinieren des ersten und zweiten Enzyms mit dem Substrat unter Bedingungen, die eine Spaltung M's von D₁ und D₂'s von D₁ bewirken;
iii) Messen der Fluoreszenzemissionsintensitäten des ersten und zweiten Farbstoffteils im Anschluss an die Kombination aus Schritt ii); und
iv) Nutzen einer Veränderung der Fluoreszenzintensitäten des ersten und zweiten Farbstoffteils, um die relativen Aktivitäten der Enzyme festzustellen.

16. Verfahren nach Anspruch 15, wobei das erste und das zweite Enzym aus jener Gruppe ausgewählt sind, die aus Phosphatase, Peptidase, Protease, Dealkylase und Glykosidase besteht.

17. Verfahren nach Anspruch 15 oder Anspruch 16, wobei der Kombinationsschritt ii) in Abwesenheit und in Anwesenheit eines Testmittels vorgenommen wird, dessen Wirkung auf die relativen Aktivitäten des ersten und zweiten Enzyms festzustellen ist; wobei ein Unterschied zwischen den relativen Aktivitäten des ersten und zweiten Enzyms in Anwesenheit und in Abwesenheit des Mittels hinweisend ist auf die Wirkung des Testmittels auf die relativen Aktivitäten des ersten und zweiten Enzyms.

18. Testkit zur Feststellung der Aktivität eines Enzyms, wobei das Kit ein oder mehrere verschiedene Enzymsubstrate umfasst, wobei jedes der Substrate eine Verbindung der Formel (I) umfasst, wie in Anspruch 1 definiert, wobei D₁, D₂, L und M definiert sind wie in Anspruch 1.

19. Testkit nach Anspruch 18, weiterhin umfassend eines oder mehrere verschiedene der Enzyme, wobei jedes Enzym spezifisch für ein anderes der Substrate ist.

## Revendications

1. Composé selon la formule (I) :
M-D₁-L-D₂ (I)
dans laquelle :
D₁ représente une première fraction colorant dont les propriétés de fluorescence peuvent être modulées de manière qu'il soit approprié en tant que donneur dans un agencement de transfert d'énergie, dans lequel D₁ est capable de transférer de l'énergie à D₂ et est sélectionné parmi les colorants coumarines, colorants benzocoumarines, colorants acridones, colorants xanthines, colorants phénoxazines, colorants rhodamines, colorants mérocyanines, et les colorants cyanines, de préférence les colorants xanthines et les colorants cyanines ;
D₂ est une seconde fraction colorant appropriée en tant que receveur dans un agencement de transfert d'énergie avec ledit premier colorant, et est sélectionné parmi : des colorants coumarines, colorants benzocoumarines, colorants acridones, colorants xanthines, colorants phénoxazines, colorants rhodamines, colorants mérocyanines et les colorants cyanines ;
M est un groupe pouvant être clivé par enzyme choisi afin de moduler les propriétés de fluorescence de D₁, dans lequel M comprend un substrat d'une première enzyme de clivage ;
L est un groupe de liaison comprenant 2 à 200 atomes liés, dans lequel ledit groupe de liaison est un lieur pouvant être clivé et comporte un groupe pouvant être clivé par enzyme P qui est un substrat pour une seconde enzyme de clivage différente de ladite première enzyme de clivage.

2. Composé selon la revendication 1, dans lequel ladite première enzyme de clivage est sélectionnée à partir du groupe constitué par une peptidase, une protéase, une phosphatase, et une déalkylase et une glycosidase.

3. Composé selon l'une ou l'autre des revendications 1 ou 2, dans lequel M représente le groupe : 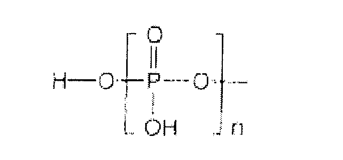 dans lequel n est un entier de 1 à 4.

4. Composé selon l'une ou l'autre des revendications 1 ou 2, dans lequel M comprend au moins une liaison peptide (-CO-NH-) liée de manière covalente à D₁.

5. Composé selon l'une ou l'autre des revendications 1 ou 2, dans lequel M comprend une liaison glycosidique qui est un substrat pour une glycosidase.

6. Composé selon l'une ou l'autre des revendications 1 ou 2, dans lequel M comprend une liaison éther qui est un substrat pour une déalkylase.

7. Composé selon l'une ou l'autre des revendications 1 ou 2, dans lequel M comprend, en outre, un groupe de perméabilisation de membrane de cellulaire.

8. Composé selon la revendication 7, dans lequel ledit groupe perméabilisation de ladite membrane de cellules est sélectionné à partir des groupes : 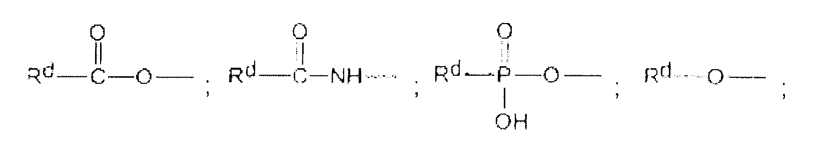 dans lesquels R^{d} représente :
une chaîne alkyle en C₁-C₁₀ droite ou ramifiée, soit non substituée soit substituée avec un ou plusieurs atomes d'halogène ;
un phényle, soit non substitué soit substitué avec un ou plusieurs atomes d'halogène ; ou
une chaîne peptide.

9. Composé selon l'une quelconque des revendications 1 à 8, dans lequel le groupe de liaison L est sélectionné à partir d'atomes de carbone qui comportent un ou plusieurs groupes sélectionnés à partir -C(O)-, -NR'-, -O-, - S-, -CH=CH-, -CO-NH-, un phénylényle et le groupe : 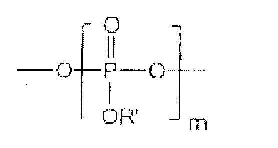 dans lequel R' est sélectionné à partir d'hydrogène et d'un alkyle en C₁-C₄ et m est un entier de 1 à 3.

10. Composé selon l'une quelconque des revendications 1 à 8, dans lequel le groupe de liaison L comprend un peptide ou un fragment d'oligonucléotide.

11. Composé selon l'une quelconque des revendications 1 à 10, dans lequel ledit colorant donneur est un colorant xanthine ou un colorant cyanine et le colorant receveur est un colorant rhodamine ou cyanine.

12. Composé selon l'une quelconque des revendications 1 à 10, dans lequel au moins l'une desdites fractions de colorant donneur et receveur est un colorant cyanine.

13. Composé selon l'une quelconque des revendications 1 à 10, dans lequel ledit colorant donneur est un colorant xanthine et ledit colorant receveur est un colorant rhodamine.

14. Composé selon l'une quelconque des revendications précédentes, comprenant, en outre, des constituants de solubilisation à l'eau fixés dessus, lesdits constituants de solubilisation à l'eau étant sélectionnés à partir du groupe constitué par un sulfonate, l'acide sulfonique, un phosphate, un phosphonate, un ammonium quaternaire et de l'hydroxyle.

15. Procédé de détermination d'activités relatives de deux enzymes agissant sur une molécule de substrat, ladite molécule de substrat comprenant un composé selon la formule I
M-D₁-L-D₂
dans laquelle les D₁, D₂, M et L sont tel que défini dans la revendication 1 ; le procédé comprenant les étapes de :
i) mesure de l'intensité d'émission de fluorescence du substrat marqué de manière fluorescente ;
ii) association desdites première et seconde enzymes avec ledit substrat sous des conditions destinées à provoquer le clivage de M par D₁ et de D₂ par D₁ ;
iii) mesure des intensités d'émission de fluorescence desdites première et seconde fractions de colorant suite à l'association de l'étape ii) ; et
iv) utilisation d'une variation sur les intensités de fluorescence desdites première et seconde fractions de colorant afin de déterminer les activités relatives desdites enzymes.

16. Procédé selon la revendication 15, dans lequel ladite première et ladite seconde enzymes sont sélectionnées à partir du groupe constitué par la phosphatase, la peptidase, la protéase, la déalkylase et la glycosidase .

17. Procédé selon la revendication 15 ou la revendication 16, dans lequel ladite étape d'association ii) est réalisée en l'absence et en la présence d'un agent de test dont l'effet sur les activités relatives desdites première et seconde enzymes doit être déterminé ; dans lequel une différence entre les activités relatives desdites première et seconde enzymes en la présence et en l'absence dudit agent est représentative de l'effet dudit agent de test sur les activités relatives desdites première et seconde enzymes,

18. Kit de test destiné à déterminer l'activité d'une enzyme, ledit kit comprenant un ou plusieurs substrats d'enzyme différents, chacun desdits substrats comprenant un composé de formule (I) selon la revendication 1, dans laquelle D₁, D₂, L et M sont définis comme dans la revendication 1.

19. Kit de test selon la revendication 18, comprenant, en outre, une ou plusieurs dites enzymes différentes, chaque enzyme étant spécifique pour un dit substrat différent.
